(19) Europäisches Patentamt
European Patent Office
Office européen des brevets

(11) Publication number: **0 116 208**
**B1**

(12) **EUROPEAN PATENT SPECIFICATION**

(45) Date of publication of patent specification: **30.03.88**

(21) Application number: **83307423.0**

(22) Date of filing: **06.12.83**

(51) Int. Cl.[4]: **C 07 D 487/14,** A 61 K 31/40, A 61 K 31/44 // (C07D487/14, 209:00, 209:00, 203:00)

(54) **Mitomycin analogues.**

(30) Priority: **07.12.82 JP 214509/82**
**25.03.83 JP 50186/83**

(43) Date of publication of application:
**22.08.84 Bulletin 84/34**

(45) Publication of the grant of the patent:
**30.03.88 Bulletin 88/13**

(84) Designated Contracting States:
**DE FR GB IT**

(56) References cited:
**FR-A-2 505 839**
**US-A-3 332 944**
**US-A-4 268 676**

**The file contains technical information submitted after the application was filed and not included in this specification**

(73) Proprietor: **KYOWA HAKKO KOGYO CO., LTD.**
**6-1, Ohte-Machi 1-chome**
**Chiyoda-ku Tokyo-to (JP)**

(72) Inventor: **Kono, Motomichi**
**2525, Shimo-oyamada-machi**
**Machida-shi Tokyo-to (JP)**
Inventor: **Saito, Yutaka**
**Kenyuryo, 3-6-6 Asahi-machi**
**Machida-shi Tokyo-to (JP)**
Inventor: **Goto, Jyoji**
**Kenyuryo, 3-6-6 Asahi-machi**
**Machida-shi Tokyo-to (JP)**
Inventor: **Shirahata, Kunikatsu**
**4-11-5, Iwato-minami**
**Komae-shi Tokyo-to (JP)**
Inventor: **Morimoto, Makoto**
**1188, Shimo-togari Nagaizumi-cho**
**Sunto-gun Shizuoka-ken (JP)**
Inventor: **Ashizawa, Tadashi**
**13-3236, Ohoka**
**Numazu-shi Shizuoka-ken (JP)**

(74) Representative: **Watkins, Arnold Jack et al**
**European Patent Attorney Frank B. Dehn & Co.**
**Imperial House 15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 116 208 B1**

Courier Press, Leamington Spa, England.

**Description**

The present invention relates to mitomycin analogues, processes for their preparation, pharmaceutical compositions containing them and their use as anti-tumour and antibacterial agents.

Certain mitomycin compounds are known as antibiotics having antibacterial and antitumour activities, and are exemplified by mitomycin A, mitomycin B, mitomycin C and porfiromycin, which are referred to in the Merck Index, 9th Edition, as well as by mitomycin D and mitomycin E which are disclosed in Japanese Patent Application as laid open to public inspection as Kokai Koho 122797/79, mitomycin F (Japanese Patent Application as laid open to public inspection as Kokai Koho 45322/80) and mitomycin J (Shirahata et. al., 23rd symposium the chemistry of natural products Nagoya 608, 1980). These compounds may be obtained by culturing a microorganism of *Streptococcus caespitosus* and have the following chemical structures:

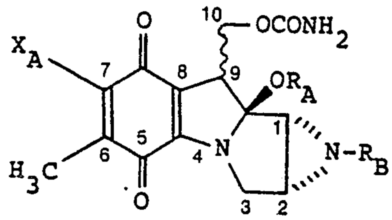

| | $X_A$ | 9 ~ 10 | $R_A$ | $R_B$ |
|---|---|---|---|---|
| Mitomycin A | $OCH_3$ | | $CH_3$ | H |
| Mitomycin B | $OCH_3$ | | H | $CH_3$ |
| Mitomycin C | $NH_2$ | | $CH_3$ | H |
| Mitomycin D | $NH_2$ | | H | $CH_3$ |
| Mitomycin E | $NH_2$ | | $CH_3$ | $CH_3$ |
| Mitomycin F | $OCH_3$ | | $CH_3$ | $CH_3$ |
| Mitomycin J | $OCH_3$ | | $CH_3$ | $CH_3$ |
| Porfiromycin | $NH_2$ | | $CH_3$ | $CH_3$ |

Various derivatives of the above-mentioned mitomycin compounds are also known. For example, certain derivatives of mitomycin C, in which the 7-amino group is substituted with various different groups have been described in U.S. Patent No. 4,268,676, in J. Med. Chem. *24,* 975 (1981) and in a Japanese Patent Application laid open to public inspection as Kokai Koho 188590/82.

US Patent 4,268,676 and J. Med. Chem., *24,* 975 (1981) disclose the presence of 2-thiazolylamino, furfurylamino, 2-propenylamino, 2-propynylamino, 2-thienylmethylamino and carbamoylmethylamino groups at the 7-position.

Japanese Patent Application as laid open to public inspection as Kokai Koho 188590/82 discloses mitomycin derivatives, in which the 7-amino group is substituted with various heterocyclic groups (quinolinyl, pyrazolyl, thiazolyl etc.), or the 7-position is substituted with various heterocyclic groups containing a nitrogen atom (1-pyrrolinyl, 1-indolinyl, etc.), the 2-mercaptoethylamino group (Examples 16 and 33) or the 2-(ethylthio)ethylamino group (Example 32).

The present invention is based on the discovery that mitomycin analogues having a 7-amino group substituted by certain groups comprising the disulfide linkage —S—S— possess good antibacterial and anti-tumour activity and have not hitherto been reported.

Thus according to one feature of the present invention there are provided compounds of the general formula:

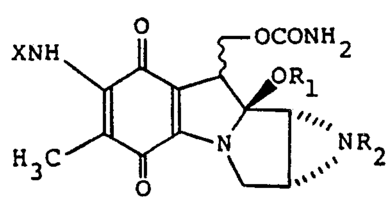

(I)

{wherein X represents a $R_3$—S—S—$CH_2$—$CH_2$— group [in which $R_3$ represents a cycloalkyl or straight chain or branched alkyl group having up to 7 carbon atoms, said alkyl or cycloalkyl group optionally carrying 1 to 3 amino groups and/or 1 to 3 hydroxyl groups; a —$(CH_3)_nCO_2R_4$ group (in which n is the integer 1, 2 or 3, and $R_4$ represents a methyl or ethyl group); or a 2-pyridyl group] and at least one of $R_1$ and $R_2$ represents a methyl group and another represents a hydrogen atom or a methyl group, and ∿∿ indicates α or β bonding}.

With regard to the definition of $R_3$ in general formula (I), the cycloalkyl or straight chain or branched alkyl groups having up to 7 carbon atoms optionally substituted by 1 to 3 hydroxyl groups and/or 1 to 3 amino groups, are exemplified by $C_{1-4}$, e.g. $C_{1-3}$, straight chain or branched alkyl groups optionally carrying 1, 2 or 3 hydroxy groups and/or 1, 2 or 3 amino groups e.g. a methyl, ethyl, n-propyl, isopropyl, n-butyl, sec-butyl, tert-butyl, 2-hydroxyethyl, 3-hydroxypropyl, 2,3-dihydroxypropyl, 2-aminoethyl, 3-aminopropyl, 2-amino-1-methylethyl or 4-aminobutyl group; or a $C_5$ or $C_6$ cycloalkyl group optionally carrying 1 to 3 hydroxy groups and/or 1 to 3 amino groups such as a cyclopentyl, cyclohexyl or 4-hydroxycyclohexyl group.

With regard to the definition of $R_3$ as a —$(CH_2)_nCO_2R_4$ group such groups may be exemplified by the ethoxycarbonylmethyl and methoxycarbonylethyl group.

The following compound is especially excellent in chemotherapeutic index on anti-tumour activity and low toxicity against bone marrow, namely, 7-N-[2-(2,3-dihydroxypropyl)dithioethyl] mitomycin C.

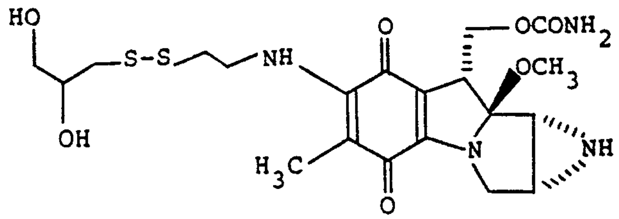

The compounds of formula I as hereinbefore defined, may, if desired, be prepared by either of the following processes, which processes constitute further features of the present invention:—

a) The reaction of a compound of the formula:—

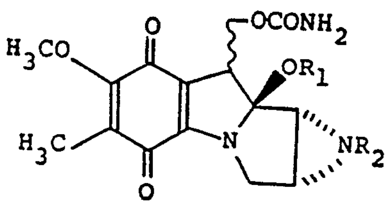

(II)

(wherein $R_1$ and $R_2$, which may be the same or different, are as hereinbefore defined) with a compound of the formula:—

$$X—NH_2 \quad (III)$$

(wherein X is as hereinbefore defined) or a salt thereof; and

b) For the preparation of compounds of formula I as hereinbefore defined [wherein X represents the group $R_3'$—S—S—$CH_2$—$CH_2$— in which $R_3'$ represents a cycloalkyl or straight chain or branched alkyl group with up to 7 carbon atoms said alkyl or cycloalkyl group optionally carrying 1 to 3 amino groups and/or 1 to 3 hydroxy groups; or $R_3'$ represents a —$(CH_2)_nCO_2R_4$ group (in which n is the integer 1, 2 or 3 and $R_4$ represents a methyl or ethyl group), and at least one of $R_1$ and $R_2$ represents a methyl group and another represents a hydrogen atom or a methyl group and ∿∿ indicates α or β bonding] the reaction of a compound of the formula:—

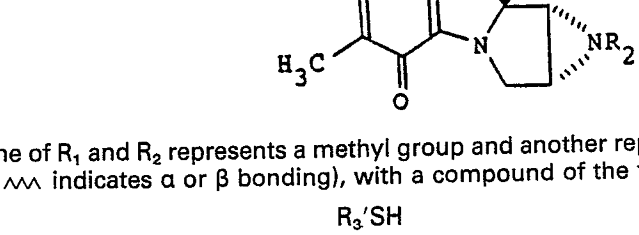

(IV)

(wherein at least one of $R_1$ and $R_2$ represents a methyl group and another represents a hydrogen atom or a methyl group and ∿∿ indicates α or β bonding), with a compound of the formula:—

$$R_3'SH \quad (V)$$

(wherein $R_3'$ is as hereinbefore defined) or, where appropriate, a base addition salt thereof.

Process (a) is conveniently effected in the presence of an inert solvent, advantageously under basic conditions (e.g. in the presence of a base) and preferably at a temperature of from 0°C to ambient temperature.

The compound of formula (III) may, if desired, be used in the form of an acid addition salt (e.g. the hydrochloride, sulfate or nitrate) and where 4-amino-1,2-dithiolane is used, conveniently the hydrochloride thereof.

Where more than 2 mols of the compound of formula III wherein $R_3$ represents a cycloalkyl or a straight chain or branched alkyl group, having 2—7 carbon atoms and substituted an amino group (hereinafter referred to as formula III') is used per mol of the compound of formula II, the resultant product is formed by reaction of the compound of formula (II) with the compound of formula (III') in a ratio of 1:1 as exemplified in Example 1 hereinafter, and where about 0.5 mol of the compound of formula (III') is used per mol of the compound of formula (II), the resultant product is obtained by reaction of the compound of formula (II) with the compound of formula (III') in a ratio of 2:1.

Methanol, ethanol, isopropanol, methylene chloride, chloroform, acetonitrile, tetrahydrofuran, dioxan and/or dimethylformamide may for example be used either alone or in combination as the inert solvent.

The reaction may for example be effected in the presence of a tertiary amine such as triethylamine or pyridine in order to ensure basic conditions.

Although the reaction time may vary with solvents used and with the compounds (II) and (III) used, the reaction is usually completed within a period of from scores of minutes to several hours, and where 4-amino-1,2-dithiolane is used, the reaction is usually complete within a period of from several hours to several days. After completion of the reaction, water may be poured into the reaction mixture which may then be extracted with for example chloroform or ethyl acetate. After concentrating the extracted solution *in vacuo*, the concentrated solution may be purified, for example by column chromatography, preparative thin layer chromatography and/or recrystallization.

The synthesis of 4-amino-1,2-dithiolane hydrochloride is described in Chemical Abstracts, *63*, 18061 (1965).

With regard to process (b) the reaction may if desired be effected using a compound of formula V in the form of a salt (e.g. a base addition salt) for example with an alkali metal or alkaline earth metal e.g. the potassium or sodium salt. The reaction is conveniently effected in the presence of an inert solvent, advantageously at a temperature of from 0°C to ambient temperature.

The reaction (b) may be effected, for example, by using as the inert solvent, methanol, ethanol, isopropanol, acetonitrile, dimethylformamide, dimethylsulfoxide or another organic solvent and/or water. These solvents may be used alone or in combination.

The reaction time may vary, depending upon the kind of the compound (V) used, although the reaction is usually complete within a period of from several minutes to several hours. After completion of the reaction, water is poured into the reaction mixture which is then extracted with chloroform or ethyl acetate. The extracted solution is conveniently concentrated *in vacuo* and, if desired, purified by, for example, column chromatography, preparative thin layer chromatography, and/or recrystallization. The synthesis of a disulfide by the use of a 2-pyridyldisulfide and its uses are described, for example, in Biochem. J., *173*, 723 (1978); Biochem Biophy. Res. Comm., *101*, 599 (1981); Cancer Res., *42*, 457 (1982) and Nature, *290*, 145 (1981).

Minimum growth inhibiting concentrations of various compounds (I) [μg/ml; agar dilution method; pH = 7.0] are shown in the following Table 1 in which the various compounds are indicated by the following abbreviations:

c. 7-N-[2-(2-aminoethyl)dithioethyl] mitomycin D

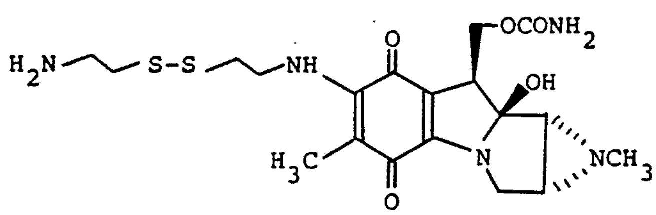

d. 7-N-[2-(2-pyridyldithio)ethyl] mitomycin C

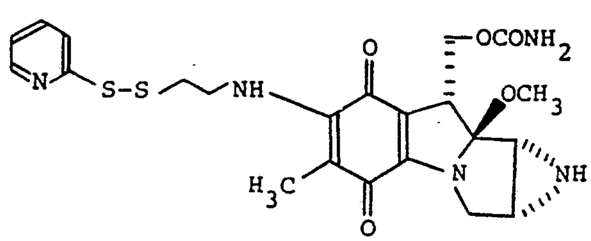

e. 7-N-[2-(2-pyridyldithio)ethyl] profiromycin

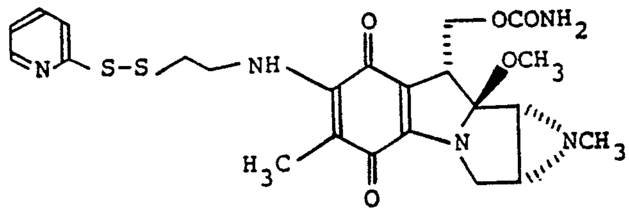

f. 7-N-[2-(2-pyridyldithio)ethyl] mitomycin D

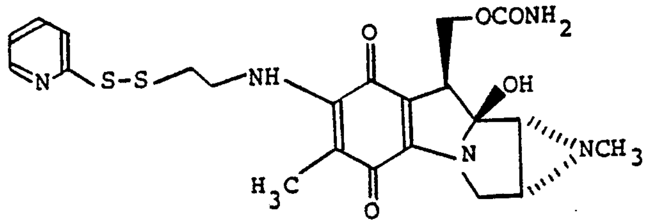

g. 7-N-(2-ethyldithioethyl)mitomycin C

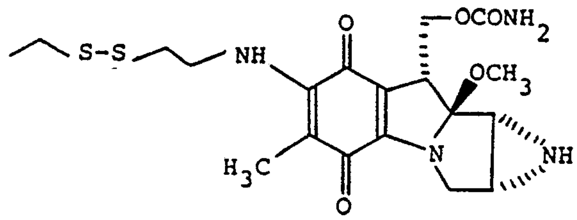

h. 7-N-(2-propyldithioethyl) mitomycin C

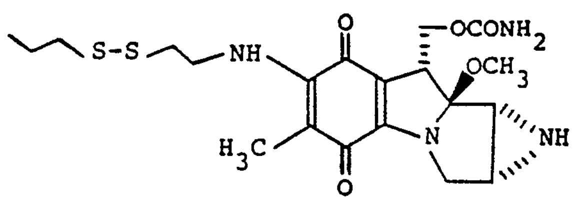

i. 7-N-[2-(2-hydroxyethyl)dithioethyl] mitomycin C

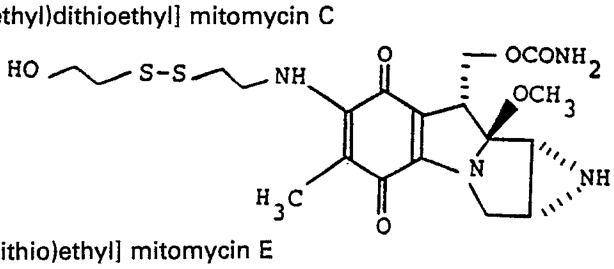

n. 7-N-[2-(2-pyridyldithio)ethyl] mitomycin E

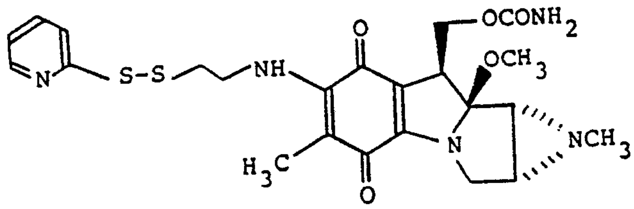

o. 7-N-[2-(2,3-dihydroxypropyl)dithioethyl] mitomycin C

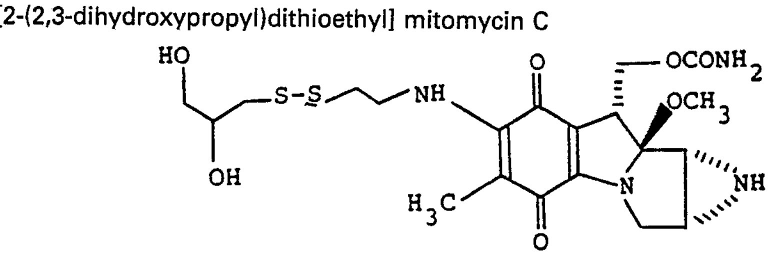

p. 7-N-(2-methoxycarbonylmethyldithioethyl) mitomycin C

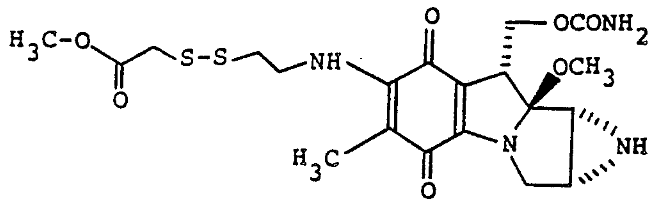

q. 7-N-(2-cyclohexyldithioethyl) mitomycin C

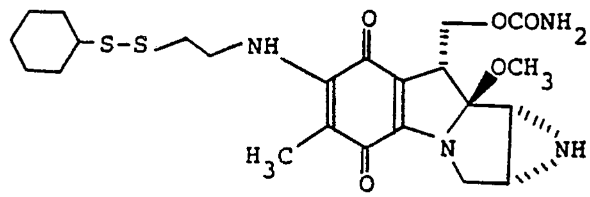

r. 7-N-[2-(2-aminoethyl)dithioethyl] mitomycin C

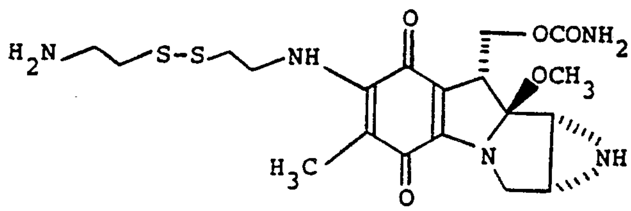

u. 7-N-[2-(2-aminoethyl)dithioethyl] profiromycin

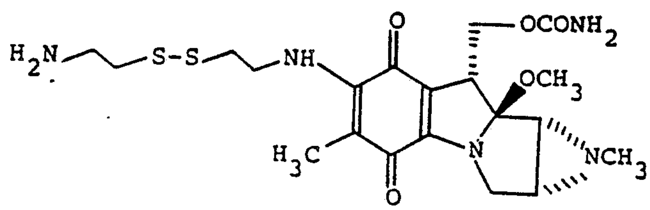

TABLE 1

A . . . Mitomycin derivatives, B . . . Microorganisms*

| A \ B | CA | SF | SA | EC | BS | PV | SS | ST | KP |
|---|---|---|---|---|---|---|---|---|---|
| c | — | 20 | 20 | — | 5 | — | — | — | — |
| d | >20 | 0.039 | 0.039 | 20 | <0.0098 | 1.3 | 10 | 20 | 0.31 |
| e | >20 | 1.3 | 0.31 | — | 0.078 | 20 | >20 | >20 | 10 |
| f | — | 1.3 | 1.3 | — | 0.31 | 10 | — | — | — |
| g | — | <0.0098 | <0.0098 | — | <0.0098 | 0.31 | >20 | — | 0.16 |
| h | — | 0.039 | 0.020 | — | <0.0098 | 0.63 | >20 | >20 | 0.63 |
| i | — | 0.039 | 0.16 | — | 0.078 | 2.5 | >20 | — | 0.31 |
| n | — | 20 | 0.63 | — | 0.078 | 10 | — | — | >20 |
| o | — | >20 | >20 | — | >20 | 20 | — | — | 2.5 |
| p | — | >20 | >20 | >20 | >20 | 2.5 | 20 | 20 | 0.16 |
| q | — | >20 | 1.3 | — | 20 | 10 | — | — | 2.5 |
| r | — | 1.3 | 0.63 | — | 0.16 | 5 | — | — | 2.5 |
| u | — | 2.5 | 0.63 | — | 0.039 | >10 | — | — | 5 |
| MM—C | — | 0.039 | 0.020 | 2.5 | <0.0049 | 0.020 | 1.3 | 1.3 | 0.0049 |

*Notes:—

| | |
|---|---|
| MM—C | Mitomycin C |
| CA | *Candida albicans* ATCC 10231 |
| SF | *Streptococcus faecalis* ATCC 10541 |
| SA | *Staphylococcus aureus* ATCC 6538P |
| EC | *Escherichia coli* ATCC 26 |
| BS | *Bacillus subtilis* 10707 |
| PV | *Proteus vulgaris* ATCC 6897 |
| SS | *Shigella sonnei* ATCC 9290 |
| ST | *Salmonella typhosa* ATCC 9992 |
| KP | *Klebsiella pneumoniae* ATCC 10031 |

---

The following examples illustrate the invention.

Example 1

Preparation of 7-N-[2-(2-aminoethyl)dithioethyl]mitomycin D

Cystamine dihydrochloride (103 mg) is dissolved in methanol (2.5 ml), to which triethylamine (0.4 ml) is then added dropwise with stirring. To the mixture is added mitomycin B (40 mg in total) in several portions, and the mixture is stirred for 40 minutes at ambient temperature. Water is added to the solution which is then extracted with chloroform. The extracted solution is dried with anhydrous sodium sulfate and the solvent is removed by distillation *in vacuo*. The resultant oily substance is purified by column chromatography using Diaion HP—20 (a resin commercially available from Mitsubishi Kasei Kogyo K.K., Tokyo. DIAION is a registered Trade Mark). The elution is effected by using a solvent system of a mixture of

water/methanol (1:4 v/v) to give active fractions which are concentrated to obtain a dark green paste (23 mg) having the following characteristics (yield 43%):—

Elemental analysis (%): as $C_{19}H_{27}N_5O_5S_2$

Found: H 5.82 C 48.67 N 14.70

Calculated: H 5.80 C 48.60 N 14.91

$^1H$—NMR spectrum (py—$d_5$): δ 2.08 (3H, s), 2.13 (3H, s), 2.23 (1H, dd), 2.47 (1H, d), 2.78—3.17 (6H, m), 3.66 (1H, d), 3.86 (2H, q), 4.21 (1H, dd), 4.43 (1H, d), 5.19 (1H, dd), 5.46 (1H, dd), 7.23 (1H, t), 7.45 (2H, br. s).

IR spectrum (KBr method): 3280, 1707, 1629, 1547, 1511, 1451, 1332, 1071 $cm^{-1}$

Example 2

Preparation of 7-N-[2-(2-pyridyldithio)ethyl]mitomycin C

50 mg of 2-pyridyldithioethylamine dihydrochloride (disclosed in Japanese Patent Application as laid open to public inspection as Kokai Koho 136,261/80) is dissolved in methanol (2.5 ml), to which triethylamine (0.2 ml) is added dropwise while stirring. Mitomycin A (40 mg in total) is added in several portions to the mixture which is then stirred at ambient temperature for 5 hours. Water is added to the reaction solution and the extraction is effected by using chloroform. The extracted solution is dried over anhydrous sodium sulfate and distilled *in vacuo* to remove the solvent. The resultant oily substance is purified by silica gel column chromatography. The elution is effected by using a solvent system of a mixture of ethyl acetate/methanol (95:5 v/v) to give active fractions which are concentrated to obtain a blackish purple powder (54 mg) having the following characteristics (yield 94%):

Elemental analysis (%): as $C_{22}H_{25}N_5O_5S_2$

Found: H 5.06 C 52.58 N 13.63

Calculated: H 5.00 C 52.47 N 13.91

IR spectrum (KBr method): 3290, 2930, 1713, 1632, 1553, 1505, 1444, 1414, 1330, 1061, 755 $cn^{-1}$

Example 3

Preparation of 7-N-[2-(2-pyridyldithio)ethyl]porfiromycin

A similar procedure to that described in Example 2 is effected by using 2-pyridyldithioethylamine dihydrochloride (50 mg) and mitomycin F (40 mg) to obtain a blackish purple powder having the following characteristics (56 mg: yield 98%):—

Elemental analysis (%): as $C_{23}H_{27}N_5O_5S_2$

Found: H 5.30 C 53.41 N 13.32

Calculated: H 5.26 C 53.37 N 13.53

$^1H$—NMR spectrum ($CDCl_3$): δ 1.94 (3H, s), 2.27 (3H, s), 2.27 (2H, m), 2.96 (2H, t), 3.18 (3H, s), 3.45 (1H, dd), 3.57 (1H, dd), 3.86 (2H, q), 4.24 (1H, d), 4.35 (1H, dd), 4.72 (1H, dd), 4.73 (2H, br. s), 7.10 (1H, br.), 7.13 (1H, m), 7.50—7.72 (2H, m), 8.59 (1H, m).

IR spectrum (KBr method): 3290, 2930, 1719, 1632, 1557, 1508, 1445, 1326, 1060, 758 $cm^{-1}$.

Example 4

Preparation of 7-N-[2-(2-pyridyldithio)ethyl]mitomycin D

A similar procedure to that described in Example 2 is carried out by using 2-pyridylthioethylamine dihydrochloride (50 mg) and mitomycin B (40 mg) to obtain 57 mg of a dark green powder having the following characteristics (yield: 99%):—

Elemental analysis (%): as $C_{22}H_{25}H_5O_5S_2$

Found: H 5.03 C 52.51 N 13.69

Calculated: H 5.00 C 52.47 N 13.91

$^1H$—NMR spectrum ($CDCl_3$): δ 1.91 (3H, s), 2.27 (3H, s), 2.27 (2H, m), 2.96 (2H, t), 3.49 (1H, br. d), 3.71 (1H, t), 3.84 (2H, q), 4.14 (1H, d), 4.48 (1H, br.), 4.72 (2H, d), 4.81 (2H, br.), 7.02 (1H, br.), 7.10 (1H, m), 7.50—7.70 (2H, m), 8.56 (1H, m).

IR spectrum (KBr method): 3270, 1713, 1631, 1601, 1548, 1510, 1450, 1414, 1332, 1113, 1058, 758 $cm^{-1}$.

Example 5

Preparation of 7-N-(2-ethyldithioethyl)mitomycin C

7-N-(2-pyridyldithioethyl)mitomycin C prepared by the method of Example 2 (50 mg) is dissolved in methanol (2.5 ml), to which ethanethiol (8 µl) is then added. The mixture is stirred at ambient temperature for 10 minutes, and a saturated aqueous solution of sodium hydrogen carbonate is poured into the mixture. The mixture is extracted with chloroform and the extracted solution is dried over anhydrous sodium sulfate. The dried material is distilled *in vacuo* to remove the solvent. The resultant oily substance is purified by silica gel column chromatography. The elution is effected by using a solvent system of a mixture of chloroform/methanol (96:4 v/v) to give active fractions which are concentrated to obtain a

residue which is then dissolved in chloroform. This solution is dropwise added to cyclohexane, from which a greyish blue powder (43 mg) having the following characteristics is recovered (yield 96%):—

Elemental analysis (%): as $C_{19}H_{26}N_4O_5S_2$
Found: H 5.79 C 50.29 N 12.16
Calculated: H 5.76 C 50.20 N 12.33

$^1$H—NMR spectrum ($CDCl_3$): δ 1.33 (3H, t), 2.03 (3H, s), 2.67 (2H, q), 2.84 (2H, t), 2.85 (2H, m), 3.21 (3H, s), 3.51 (1H, dd), 3.62 (1H, dd), 3.87 (2H, q), 4.28 (1H, d), 4.50 (1H, dd), 4.68 (2H, br. s), 4.71 (1H, dd), 6.49 (1H, br. t).

IR spectrum (KBr method): 3270, 1718, 1633, 1554, 1507, 1447, 1328, 1061 $cm^{-1}$.

## Example 6

### Preparation of 7-N-(2-propyldithioethyl)mitomycin C

7-N-(2-pyridyldithioethyl) mitomycin C (50 mg) and propanethiol (9 µl) are treated in a similar manner to that described in Example 5 to obtain 29 mg of a greyish blue powder having the following characteristics with a yield of 62%:—

Elemental analysis (%): as $C_{20}H_{28}N_4O_5S_2$
Found: H 6.03 C 51.32 N 11.75
Calculated: H 6.02 C 51.26 N 11.96

$^1$H—NMR spectrum ($CDCl_3$): δ 0.99 (3Ha, t), 1.71 (2H, sextet), 2.03 (3H, s), 2.68 (2H, t), 2.83 (2H, t), 2.85 (2H, m), 3.21 (3H, s), 3.51 (1H, dd), 3.60 (1H, dd), 3.84 (2H, q), 4.28 (1H, d), 4.50 (1H, dd), 4.68 (2H, br. s), 4.71 (1H, dd), 6.48 (1H, br. t).

IR spectrum (KBr method): 3290, 1721, 1634, 1558, 1509, 1448, 1327, 1060 $cm^{-1}$.

## Example 7

### Preparation of 7-N-[2-(2-hydroxyethyl)dithioethyl]mitomycin C

A similar procedure to that described in Example 5 is carried out by using 7-N-(2-pyridyldithioethyl) mitomycin C (50 mg) and 2-mercaptoethanol (7 µl) to obtain a greyish blue powder (41 mg) having the following characteristics with a yield of 88%:—

Elemental analysis (%): as $C_{19}H_{26}N_4O_6S_2$
Found: H 5.60 C 48.12 N 11.62
Calculated: H 5.57 C 48.50 N 11.91

$^1$H—NMR spectrum ($CDCl_3$): δ 2.02 (3H, s), 2.80—2.97 (2H, m), 2.86 (2H, t), 2.90 (2H, t), 3.21 (3H, s), 3.51 (1H, dd), 3.59 (1H, dd), 3.87 (2H, q), 3.88 (2H, t), 4.27 (1H, d), 4.50 (1H, dd), 4.70 (1H, dd), 4.78 (2H, br. s), 6.49 (1H, br. t).

IR spectrum (KBr method): 3430, 3280, 1712, 1632, 1551, 1510, 1448, 1328, 1060 $cm^{-1}$.

## Example 8

### Preparation of 7-N-[2-(2-pyridyldithio)ethyl]mitomycin E

A similar procedure to that described in Example 2 is carried out by using 2-pyridyldithioethylamine dihydrochloride (50 mg) and mitomycin J (40 mg) to obtain a blackish purple powder (55 mg) having the following characteristics with a yield of 96%:—

Elemental analysis (%): as $C_{23}H_{27}N_5O_5S_2$
Found: H 5.28 C 53.47 N 13.26
Calculated: H 5.26 C 53.37 N 13.53

$^1$H—NMR spectrum ($CDCl_3$): δ 1.93 (3H, s), 2.21 (1H, d), 2.32 (3H, s), 2.37 (1H, dd), 2.96 (2H, t), 3.31 (3H, s), 3.57 (1H, dd), 3.81 (1H, dd), 3.85 (2H, q), 4.02 (1H, d), 4.45 (1H, dd), 4.66 (2H, br. s), 4.82 (1H, dd), 6.99 (1H, br. t), 7.11 (1H, m), 7.58 (2H, m), 8.57 (1H, m).

IR spectrum (KBr method): 3280, 2950, 1717, 1631, 1551, 1507, 1445, 1413, 1332, 1112, 1047, 757 $cm^{-1}$.

## Example 9

### Preparation of 7-N-[2-(2,3-dihydroxypropyl)dithioethyl]mitomycin C

A similar procedure to that described in Example 5 is carried out by using 7-N-(2-pyridyldithioethyl) mitomycin C (40 mg) and 1-mercapto-2,3-propanediol (7.1 µl) to obtain a black paste, which is then freeze-dried to obtain a black powder having the following characteristics (33 mg; yield 83%):—

Elemental analysis (%): as $C_{20}H_{28}N_4O_7S_2$
Found: H 5.62 C 47.96 N 11.01
Calculated: H 5.64 C 47.99 N 11.19

IR spectrum (KBr method): 3430, 3280, 2930, 1701, 1632, 1553, 1510, 1449, 1332, 1064, 755 $cm^{-1}$.

Example 10

Preparation of 7-N-(2-methoxycarbonylmethyldithioethyl)mitomycin C

A similar procedure to that described in Example 5 is carried out by using 7-N-(2-pyridyldithioethyl) mitomycin C (50 mg) and methyl thioglycolate (8.8 µl) to obtain a greyish blue powder (34 mg) having the following characteristics with a yield of 69%:—

Elemental analysis (%): as $C_{20}H_{26}N_4O_7S_2$

Found: H 5.28 C 48.21 N 10.97

Calculated: H 5.26 C 48.18 N 11.24

$^1$H—NMR spectrum ($CDCl_3$): δ 2.02 (3H, s), 2.84 (2H, br. s), 2.95 (2H, t), 3.21 (3H, s), 3.51 (2H, s), 3.52 (1H, br. d), 3.60 (1H, dd), 3.76 (3H, s), 3.89 (2H, q), 4.28 (1H, d), 4.50 (1H, dd), 4.73 (1H, dd), 4.74 (2H, br. s), 6.47 (1H, br. t).

IR spectrum (KBr method): 3280, 2930, 1723, 1554, 1508, 1445, 1330, 1060 $cm^{-1}$.

Example 11

Preparation of 7-N-(2-cyclohexyldithioethyl)mitomycin C

7-N-(2-pyridyldithioethyl) mitomycin C (50 mg) is dissolved in methanol (2.5 ml), to which is then added cyclohexanethiol (12.1 µl). The mixture is stirred at ambient temperature for 12 hours under nitrogen stream and is then treated in a similar manner to that described in Example 5 to obtain a greyish blue powder (49.5 mg) having the following characteristics with a yield of 98%:—

Elemental analysis (%): as $C_{23}H_{32}N_4O_5S_2$

Found: H 6.33 C 54.33 N 10.76

Calculated: H 6.34 C 54.31 N 11.01

$^1$H—NMR spectrum ($CDCl_3$): δ 1.02—2.02 (10H, m), 2.03 (3H, s), 2.68 (1H, br.), 2.82 (4H, m), 3.20 (3H, s), 3.51 (1H, dd), 3.61 (1H, dd), 3.86 (2H, q), 4.28 (1H, d), 4.50 (1H, dd), 4.72 (1H, dd), 4.83 (2H, br. s), 6.49 (1H, br. t).

IR spectrum (KBr method): 3300, 2930, 2850, 1718, 1633, 1558, 1509, 1448, 1329, 1062 $cm^{-1}$.

Example 12

Preparation of 7-N-[2-(2-aminoethyl)dithioethyl]mitomycin C

Cystamine dihydrochloride (103 mg) is dissolved in methanol (2.5 ml), to which triethylamine (0.4 ml) is added dropwise with stirring, followed by addition of mitomycin A (40 mg in total) in several portions. After stirring for 40 minutes at ambient temperature, water is added to the reaction mixture, followed by extraction with chloroform. Anhydrous sodium sulfate is used to dry the extracted solution, from which the solvent is then removed by distillation *in vacuo*. The resultant oily substance is purified by column chromatography using Diaion HP—20. The elution is effected by using a mixture of water/methanol (1:4 v/v) to give the active fractions which are then concentrated to obtain a blackish purple paste (21 mg) having the following physical characteristics (yield 41%):—

Elemental analysis (%): as $C_{19}H_{27}N_5O_5S_2$

Found: H 5.86 C 48.72 N 14.66

Calculated: H 5.80 C 48.60 N 14.91

$^1$H—NMR spectrum (δ, py-$d_5$): 2.14 (3H, s), 2.76 (1H, br), 2.87—3.19 (7H, m), 3.22 (3H, s), 3.59 (1H, br. d), 3.85—4.06 (3H, m), 4.52 (1H, d), 5.03 (1H, t), 5.38 (1H, dd), 7.31 (1H, t), 7.58 (2H, br).

IR spectrum (KBr method): 3280, 2920, 1712, 1632, 1552, 1508, 1449, 1330, 1061 and 752 $cm^{-1}$.

Example 13

Preparation of 7-N-[2-(2-aminoethyl)dithioethyl]porfiromycin

To cystamine dihydrochloride (100 mg) dissolved in methanol (2.5 ml), triethylamine (0.4 ml) is added with stirring. Mitomycin F (40 mg in total) is added in several portions to this solution and the mixture is stirred at room temperature for 15 minutes. After this, water is added to the reaction mixture which is extracted with chloroform. Anhydrous sodium sulfate is used to dry the extracted solution, from which the solvent is then removed by distillation *in vacuo*. The resultant oily substance is purified by silica gel column chromatography. The elution is effected by using a solvent system of chloroform/methanol (3:2 v/v) to give the active fractions which are concentrated to yield a black paste (12 mg) having the following characteristics (yield, 23%):—

Elemental analysis (%): as $C_{20}H_{29}N_5O_5S_2$

Found: H 6.07 C 49.76 N 14.23

Calculated: H 6.04 C 49.67 N 14.48

$^1$H—NMR spectrum (py-$d_5$): δ 2.14 (3H, s), 2.16 (1H, m), 2.24 (3H, s), 2.53 (1H, d), 2.80—3.19 (6H, m), 3.19 (3H, s), 3.52 (1H, dd);, 3.94 (1H, dd), 3.94 (2H, q), 4.45 (1H, d), 4.77 (1H, dd), 5.31 (1H, dd), 7.27 (1H, br. t), 7.64 (2H, br. s)

IR spectrum (KBr method): 3360, 1714, 1633, 1554, 1510, 1459, 1328, 1062 $cm^{-1}$.

According to a further feature of the present invention, there is provided a pharmaceutical composition, comprising as active ingredient 7-N-[2-(2,3-dihydroxypropyl)dithioethyl] mitomycin C in association with a physiologically acceptable carrier or excipient. The carrier or excipient may take various forms depending upon the desired formulation. Thus, for example, 7-N-[2-(2,3-dihydroxypropyl)dithioethyl] mitomycin C may, for example, be dissolved in a physiological solution of sodium chloride, or a solution of glucose, lactose and/or mannitol in order to formulate a pharmaceutical composition suitable for injection.

Alternatively, it is possible to freeze-dry 7-N-[2-(2,3-dihydroxypropyl)dithioethyl]mitomycin C, to which sodium chloride is added to prepare a powdery injection agent according to the Pharmacopoeia of Japan. The composition may, if desired, comprise conventional additives or excipients such as for example pharmaceutically acceptable salts which are well known in the pharmaceutical art. The dosage units may contain any appropriate amount of active ingredient, for example, an amount of 0.02 to 1 mg/kg/day, although the daily dose may vary with differing conditions. The administration may be effected, for example, by intravenous injection and may be administered, for example, from 1 to 3 times per week. The present invention thus includes compositions of the present invention in sterile form.

If desired, oral administration may be possible. Dosage unit forms suitable for oral administration may, for example, include tablets, powders, granules and ampoules and may further contain appropriate excipients well known in the parmaceutical art. If desired, the composition may be administered, for example, into the artery, abdominal cavity or thorax.

The active compound of the present invention may be formulated as illustrated in Examples A, B and C of EP—A—116 208.

The following experiment illustrates the antitumour activities, acute toxicities ($LD_{50}$) and toxicities against bone marrow of certain compounds of the present invention:—

Experiment

(1) Effect on Sarcoma 180 solid tumour:

$5 \times 10^6$ cells of Sarcoma 180 solid tumour were inoculated into the abdominal cavities of groups of ddy mice, each group consisting of 5 mice. 7 days after this, on each occasion, the tumour cells were collected from the ascites of the mouse. The cells were washed with a sterilized physiological sodium chloride solution and used to prepare a cell suspension containing $5 \times 10^7$ cells per ml of a sterilized physiological solution of sodium chloride, of which 0.1 ml was injected under the skin of the right armpit of a ddy male mouse (body weight $20 \pm 2$ g). A test compound was dissolved in a physiological solution of sodium chloride or a similar solution containing Tween 80 (a surfactant commercially available from Atlas Chemical Industries, Inc., U.S.A., registered Trade Mark). 24 hours after the implantation of the tumours cells, the test compound (0.1 to 0.2 ml) was injected into the test mice intraperitoneally. In order to determine the antitumour activity of the test compound, 7 days after the implantation of the tumour cells, on each occasion, the major axis (a) and minor axis (b) of the tumour were measured to calculate the volume of the tumour ['a' × 'b'/2]. The anti-tumour effect was evaluated by the ratio of the tumour volume in the test animals (T) to the corresponding volume of the control (untreated) animals (C).

(2) Determination of $ED_{50}$:

A dose capable of reducing the volume of a Sarcoma 180 solid tumour to 50% of the corresponding volume of the untreated group on average was defined as the $ED_{50}$. T/C ratios and the doses were respectively plotted on the ordinate in arithmetic scale and abscissa in logarithmic scale on graph paper, from which the relationship between the doses and the T/C ratios was calculated as a straight line by the least square method. From the straight tropic line thus-obtained, the dose corresponding to a T/C of 0.5 was determined.

(3) Acute toxicity ($LD_{50}$):

On each occasion, the test compound was given to the test animal. After this, the death ratio of the animals of the test group was observed for 14 days, from which $LD_{50}$ was calculated by Behrens-Körber's method.

(4) Chemotherapeutic Index (C.I.):

The ratio of $LD_{50}$ to $ED_{50}$ was defined as C.I. A greater C.I. suggests a greater effective range of the drug.

(5) Effect upon the number of peripheral leukocytes:

$5 \times 10^6$ cells of Sarcoma 180 solid tumour were implanted under the skin of each mouse at the right armpit. 24 hours after this, venous blood (0.02 ml) was collected from the eyepit of the mouse and suspended in a Cell-Kit Seven solution (10 ml; commercially available from Toa Iryo Denshi K.K., Japan).

To the suspension, a drop of saponin solution was added to dissolve the red cells and the number of leukocytes were counted using a microcell counter. The number of leukocytes and the corresponding dose were respectively plotted on the ordinate in arithmetic scale and on the abscissa in logarithmic scale on graph paper to investigate the relationship between dosage and the number of peripherical leukocytes. A

dose capable of giving 4000 leukocytes/$mm^3$ was defined as WBC 4000, which corresponded to about ½ the number of peripheral leukocytes in normal mice.

(6) WBC 4000/$ED_{50}$:

The greater the ratio of WBC 4000 to $ED_{50}$ the smaller the decrease in the number of peripheral leukocytes viz. the lower the toxicity against bone marrow in the area of the effective dose.

The results obtained from the experiments are shown in the following Table 2.

TABLE 2

A . . . Anti-tumour activity B . . . Toxicity against bone marrow

| | | A | | B | |
|---|---|---|---|---|---|
| Compound | $LD_{50}$ | C.I. | *1 | WBC 4000/ $ED_{50}$ | *2 |
| c | 74.3 | 3.11 | 1.5 | 1.87 | 3.1 |
| g | 5.4 | 1.64 | 0.8 | 3.76 | 4.7 |
| i | ≦11.25 | ≦2.12 | ≦1 | 2.13 | 2.7 |
| o | 22.5 | — | 1.4 | — | 3.9 |
| p | 25—12.5 | — | 1.6—0.9 | — | 3.2 |
| r | 13.5 | 0.73 | 0.6 | 1.25 | 3.0 |

Notes:— *Comparison with mitomycin C
*1 . . . C.I./C.I. of mitomycin C
*2 . . . [WBC 4000/$ED_{50}$]/[WBC 4000/$ED_{54}$ of mitomycin C].

**Claims**

1. 7-N-[2-(2,3-dihydroxypropyl)dithioethyl] mytomycin C.

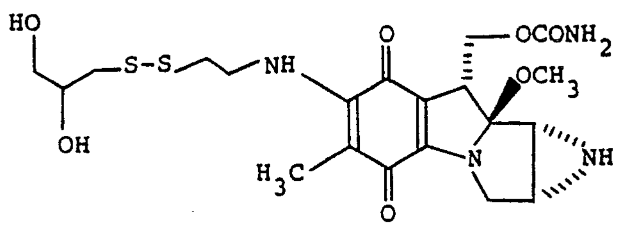

2. A pharmaceutical composition, comprising as active ingredient 7-N-[2-(2,3-dihydroxypropyl)dithioethyl] mitomycin C as claimed in claim 1 in association with a physiologically acceptable carrier or excipient.

3. Compounds of the formula:—

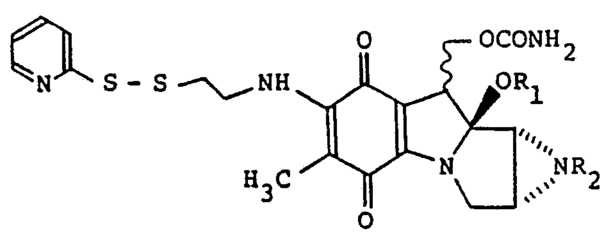

wherein at least one of $R_1$ and $R_2$ represents a methyl group and another represents a hydrogen atom or a methyl group, and ∿∿ indicates α or β bonding.

4. A process for the preparation of compounds of general formula:—

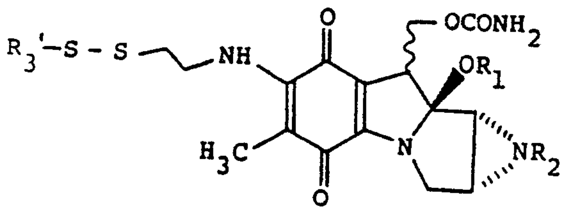

[wherein $R_3'$ represents a cycloalkyl or straight chain or branched alkyl group having up to 7 carbon atoms, said alkyl or cycloalkyl group optionally carrying 1 to 3 amino groups and/or 1 to 3 hydroxyl groups; or a —$(CH_2)_nCO_2R_4$ group (in which n is the integer 1, 2 or 3, and $R_4$ represents a methyl or ethyl group); and at least one of $R_1$ and $R_2$ represents a methyl group and another represents a hydrogen atom or a methyl group, and ∿∿ indicates α or β bonding] which comprises reacting a compound as claimed in claim 3 with a compound of formula $R_3'SH$ (wherein $R_3'$ is as defined above) or a base addition salt thereof.

5. A compound as claimed in claim 1 for use as an anti-tumour agent.

6. A compound as claimed in claim 1 for use as an anti-bacterial agent.

**Patentansprüche**

1. 7-N-[2-(2,3-dihydroxypropyl)dithioethyl]-Mitomycin C

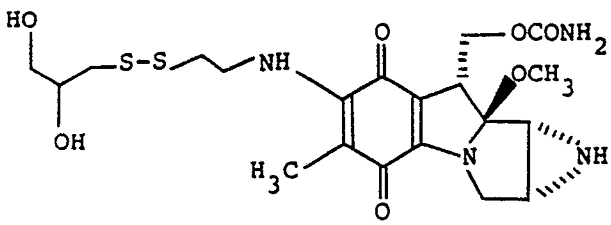

2. Pharmazeutisches Mittel, das als Wirkstoff 7-N-[2-(2,3-dihydroxypropyl)dithioethyl]-Mitomycin C gemäß Anspruch 1 zusammen mit einem physiologisch verträglichen Träger oder Exzipienten enhält.

3. Verbindungen der Formel:

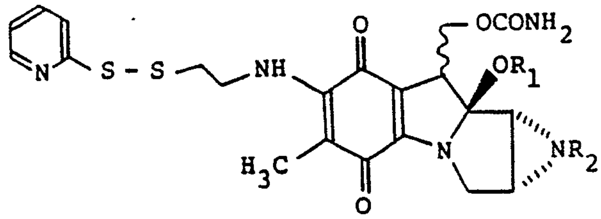

worin mindestens einer der Reste $R_1$ und $R_2$ eine Methylgruppe, und der andere Rest ein Wasserstoffatom oder eine Methylgruppe bedeuten, und ∿∿ eine α- oder β-Bindung angibt.

4. Verfahren zur Herstellung von Verbindungen der allgemeinen Formel:

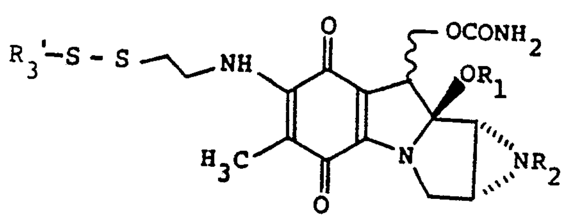

worin $R_3'$ eine Cycloalkyl- oder eine gerade oder verzweigte Alkylgruppe mit bis zu 7 Kohlenstoffatomen, wobei die Alkyl- oder Cycloalkylgruppe gegebenenfalls 1 bis 3 Aminogruppen und/oder 1 bis 3 Hydroxylgruppen besitzen kann; oder eine —$(CH_2)_nCO_2R_4$-Gruppe bedeutet, worin n für 1, 2 oder 3 steht und $R_4$ eine Methyl- oder Ethylgruppe bedeutet; mindestens einer der Reste $R_1$ oder $R_2$ eine Methylgruppe und der andere Rest ein Wasserstoffatom oder eine Methylgruppe bedeutet; und ∿∿ eine α- oder β-Bindung angibt, wobei man eine Verbindung nach Anspruch 3 mit einer Verbindung der Formel $R_3'SH$ (worin $R_3'$ wie oben definiert ist) oder einem Baseadditionssalz davon umsetzt.

5. Verwendung einer Verbindung nach Anspruch 1 als Antitumormittel.

6. Verwendung einer Verbindung nach Anspruch 1 als antibakterielles Mittel.

**Revendications**

1. La 7-N-[2-(2,3-dihydroxypropyl)dithioéthyl]mitomycine C.

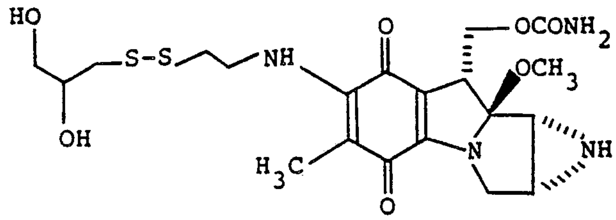

2. Composition pharmaceutique, comprenant comme ingrédient actif de la 7-N-[2-(2,3-dihydroxypropyl)dithioéthyl]mitomycine C suivant la revendication 1 en association avec un support ou excipient physiologiquement acceptable.

3. Composés de formule

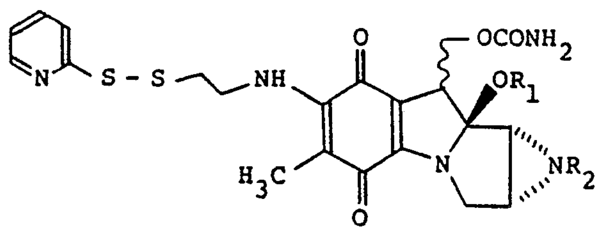

dans laquelle l'un au moins de $R_1$ et $R_2$ représente un groupe méthyle et l'autre représente un atome d'hydrogène ou un groupe méthyle, et ∿∿ indique une liaison en α ou β.

4. Procédé de préparation de composés de formule générale

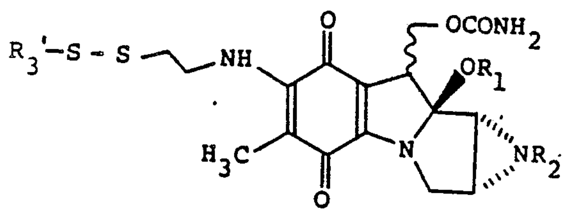

[dans laquelle $R_3'$ représente un groupe cycloalkyle ou un groupe alkyle à chaîne droite ou à chaîne ramifiée ayant jusqu'à 7 atomes de carbone, ledit groupe alkyle ou cycloalkyle portant facultativment 1 à 3 groupes amino et/ou 1 à 3 groupes hydroxyle; ou un groupe $—(CH_2)_nCO_2R_4$ (dans lequel n est le nombre entier 1, 2 ou 3 et $R_4$ représente un groupe méthyle ou éthyle); et l'un au moins de $R_1$ et $R_2$ représente un groupe méthyle et l'autre représente un atome d'hydrogène ou un groupe méthyle, et ∿∿ indique la liaison en α ou β] qui consiste à faire réagir un composé suivant la revendication 3 avec un composé de formule $R_3'SH$ (dans laquelle $R_3'$ à la définition donnée ci-dessus) ou un sel d'addition de base de ce composé.

5. Composé suivant la revendication 1, destiné à être utilisé comme agent antitumoral.

6. Composé suivant la revendication 1, destiné à être utilisé comme agent antibactérien.